# EUROPÄISCHE PATENTSCHRIFT

(11) **EP 2 976 649 B1**
(45) Veröffentlichungstag und Bekanntmachung des Hinweises auf die Patenterteilung: **23.08.2017**
(21) Anmeldenummer: 14711738.6
(22) Anmeldetag: 19.03.2014
(51) Int. Cl.: G01N 33/82

(54) **VERFAHREN UND REAGENZ ZUR BESTIMMUNG VON VITAMIN D METABOLITEN**
METHOD AND REAGENT FOR DETERMINING VITAMIN D METABOLITES
PROCÉDÉ ET RÉACTIF DE DÉTERMINATION DE MÉTABOLITES DE LA VITAMINE D

(30) Priorität: 21.03.2013 DE 102013205055
(43) Veröffentlichungstag der Anmeldung: 27.01.2016
(73) Patentinhaber: Orgentec Diagnostika GmbH, 55129 Mainz (DE)
(72) Erfinder: POPPE, Robert, 55128 Mainz (DE); SOSKIC, Vukic, 55131 Mainz (DE)
(74) Vertreter: Weiss, Wolfgang
(86) Internationale Anmeldenummer: PCT/EP2014/055503
(87) Internationale Veröffentlichungsnummer: WO 2014/147121

(56) Entgegenhaltungen:
- WO-A1-2008/092917
- DATABASE WPI Week 201143 Thomson Scientific, London, GB; AN 2011-E01872 XP002726076, & CN 101 973 913 A (WANG Y) 16. Februar 2011 (2011-02-16)
- "Iron(III) p-toluenesulfonate", INTERNET CITATION, 9. August 2005 (2005-08-09), Seiten 1-4, XP007922743, Gefunden im Internet: URL:http://pubchem.ncbi.nlm.nih.gov/summar y/summary.cgi?cid=173580 [gefunden am 2014-06-20]
- B.K ROY ET AL: "Functions of hydrotropes (sodium salicylate, proline, pyrogallol, resorcinol and urea) in solution with special reference to amphiphile behaviors", COLLOIDS AND SURFACES A: PHYSICOCHEMICAL AND ENGINEERING ASPECTS, Bd. 203, Nr. 1-3, 1. April 2002 (2002-04-01), Seiten 155-166, XP55124142, ISSN: 0927-7757, DOI: 10.1016/S0927-7757(01)01099-8
- SALEH A M ET AL: "Study of the interaction of menadione with hydrotropic salts", DIE PHARMAZIE, GOVI VERLAG PHARMAZEUTISCHER VERLAG GMBH, ESCHBORN, DE, Bd. 29, Nr. 8, 1. August 1974 (1974-08-01) , Seiten 525-527, XP008170008, ISSN: 0031-7144

## Beschreibung

Die vorliegende Erfindung betrifft ein Verfahren zur Freisetzung von gebundenem Vitamin D, indem eine Vitamin D-enthaltende Probe in Kontakt mit einem Freisetzungsreagenz gebracht wird, welches mindestens eine hydrotrope Substanz und mindestens ein Übergangsmetallsalz enthält. Das freigesetzte Vitamin D kann anschließend quantitativ bestimmt werden. Die vorliegende Erfindung betrifft weiterhin ein Reagenz zur Freisetzung und gegebenenfalls Bestimmung von Vitamin D, enthaltend mindestens eine hydrotrope Substanz und mindestens ein Übergangsmetallsalz, sowie die Verwendung eines solchen Freisetzungsreagenzes zur Freisetzung und gegebenenfalls Bestimmung von Vitamin D.

Für den Menschen ist eine ausreichende Versorgung mit Vitamin D essentiell. Die häufigsten physiologischen Formen von Vitamin D sind Vitamin D3 oder Vitamin D2. Die wichtigsten physiologisch wirksamen Derivate von Vitamin D3 (Cholecalciferol) sind 25OHD3 (25-Hydroxyvitamin D3 oder Calcidiol) und 1,25-(OH)₂ D3 (1-alpha, 25-Dihydroxyitamin D3 oder Calcitriol). In der Regel wird die überwiegende Menge Vitamin D3 nicht wie andere Vitamine mit der Nahrung aufgenommen, sondern unter dem Einfluss von UV-Licht in der Haut hergestellt. Hierbei wird 7-Dihydroxycholesterol durch UV-Bestrahlung zu Prävitamin D3 umgewandelt, wobei sich das instabile Zwischenprodukt zu Vitamin D3 umstrukturiert. In der Nahrung ist Vitamin D3 besonders in fettreichem Fisch enthalten. Eine weitere Form von Vitamin D, die im Körper vorkommen kann, ist Vitamin D2 (Calciferol oder Ergocalciferol), welches in Pilzen vorkommt und mit der Nahrung aufgenommen werden kann. Vitamin D3 und Vitamin D2 Präparate stellen eine weitere Quelle zur Versorgung mit einer ausreichenden Vitamin D Menge dar.

Vitamin D und seine natürlichen Derivate sind äußerst hydrophobe Moleküle. Im Blut erfolgt der Transport von Vitamin D im Komplex mit Vitamin D-Binde-Protein (DBP). DBP gehört zur Familie der Albumine und bindet Vitamin D, Vitamin D Metaboliten und Fettsäuren.

In der Leber wird Vitamin D durch Cytochrom P450 an Position C-25 oxidiert. Das resultierende 25OHD (25-Hydroxyvitamin D3 oder 25-Hydroxyvitamin D2) ist der hauptsächlich in der Zirkulation vorliegende Vitamin D Metabolit. Die Konzentration von 25OHD in Serum dient als Indikator zur Bewertung des Vitamin D Status des Menschen.

In der Niere und in weiteren Geweben erfolgt die Umsetzung von 25OHD zu 1,25(OH)₂D, der physiologisch aktiven Form des Vitamin D, welche nach Bindung an den Vitamin D Rezeptor (VDR) Vitamin D-abhängig regulierte (VDRE) Genaktivitäten steuert.

Eine ausreichende Versorgung mit Vitamin D ist für den normalen Knochenaufbau und die Regulation der Calcium- und Phosphatresorption wesentlich. Die Aktivierung des VDR mit 1,25(OH)₂D steigert die Effizienz der intestinalen Calciumaufnahme um ca. 30 % und die Phosphataufnahme um ca. 80 %. Wenn die Serumkonzentration von 25OHD unter 30 ng/ml absinkt, ist dies mit einer deutlichen Verringerung der Calciumresorption im Darm und einem Anstieg der Parathyroidhormonkonzentration verbunden. Vitamin D Mangel gilt als wesentlicher Faktor für Osteoporose mit erhöhtem Knochenbruchrisiko und ist mit weiteren Gesundheitsrisiken verbunden. Während die Vitamin D Konzentration im Serum abhängig von der Nahrungsaufnahme und der Sonnenexposition starken Schwankungen unterliegt, gilt die 250HD Serumkonzentration als ein guter Indikator zur Ermittlung des Vitamin D Versorgungsstatus. Die Bestimmung der 25OHD Konzentration in Serum erfolgt daher in der medizinischen Diagnostik routinemäßig.

Die Bindung von 250HD und anderer Vitamin D Substanzen an DBP stellt eine große Hürde bei der Bestimmung von Vitamin D Metaboliten dar. Alle Bestimmungsmethoden setzen eine Freisetzung des Vitamin D Metaboliten aus dem DBP-Komplex voraus.

Um Vitamin D und seine Metaboliten im Serum bestimmen zu können, müssen diese aus der Bindung mit DBP freigesetzt werden. Dies kann durch verschiedene Verfahren, wie z.B. die Ausfällung von DBP mit organischen Lösungsmitteln (z.B. Ethanol, Methanol) oder die Extraktion von Vitamin D und deren Metaboliten aus der wässrigen Phase mit phasenbildenden Lösungsmitteln, wie z.B. Chloroform oder Hexan, erfolgen.

US 7,482,162 B2 (Immunodiagnostic Systems Ltd.) beschreibt die Verwendung von 8-Anilino-1-naphthalinsulfonsäureammmoniumsalz (8-ANS) als nichtkompetitives Verdrängungsreagenz zur Freisetzung von an DBP gebundenem 1,25(OH)2D.

WO 2011/144661 (Roche Diagnostics GmbH) beschreibt eine Reagenzmischung, welche Carbonate oder Hydrogencarbonat freisetzende Substanzen in Kombination mit Reduktionsmitteln und alkalisierenden Substanzen enthält, wobei die Freisetzung von Vitamin D Metaboliten aus dem DBP-Komplex im pH-Bereich 11-14 erfolgt.

EP 2 126 586 (Immundiagnostik AG) betrifft die Anwendung eines proteolytischen Verfahrens unter Verwendung von Proteinase K zur Inaktivierung des DBP der daraus resultierenden Freisetzung von Vitamin D Metaboliten und der anschließenden Bestimmung von Vitamin D Metaboliten in einem ELISA-Testsystem. Zur Freisetzung von Vitamin D wird ein Freisetzungsreagenz verwendet, das z. B. Toluolsulfonsäure oder ein Salz der Salicylsäure umfasst, aber kein Übergangsmetallsalz.

US 7,087,395 (Quest) betrifft ein Verfahren zur Freisetzung von Vitamin D Metaboliten von DBP durch Vorbehandlung des Serums bei pH 13 mit einer alkalischen Reagenzmischung bestehen aus NaOH, Detergenzien, Cylodextrinderivaten und Salicylsäuresalzen.

WO 2008/138783 (Nordic Biosciences A/S) beschreibt die Vorbehandlung einer Serum- oder Plasmaprobe mit Pamoasäure zur Freisetzung von Vitamin D aus dem DBP-Komplex.

Die Database WPI & CN 101 973 913 A bezieht sich auf ein Herstellungsverfahren von Eisentoluolsulfonat. Zu diesem Zweck wird Eisen(III)-Nitrat mit einer wässrigen Lösung von Toluolsulfonsäure gerührt.

Bisher bekannte Methoden zur Freisetzung von Vitamin D aus dem Komplex mit DBP sind jedoch entweder arbeitsaufwendig und schwer automatisierbar oder fehleranfällig.

Automatisierbare Testverfahren zur Bestimmung von Vitamin D erfordern eine Freisetzung aus dem Komplex mit DBP unter Bedingungen, die idealerweise eine direkte weitere Analyse des Reaktionsansatzes erlauben. DBP ist jedoch ein sehr stabiles Protein und liegt in relativ hoher Serumkonzentration vor, die einer Bindekapazität von 1900 ng/ml für Vitamin D entspricht. Dies stellt, bei einer durchschnittlichen 25OHD-Serumkonzentration von 30 ng/ml, einen 60-fachen Überschuss an Bindekapazität für 25OHD dar.

Ein Freisetzungsreagenz sollte also Vitamin D aus der stabilen Bindung mit DBP lösen, den erheblichen Überschuss an Vitamin D Bindekapazität, der durch die hohe DBP-Serumkonzentration vorliegt, inaktivieren und zusätzlich die Bindung des freigesetzten Vitamin D an ein Detektionsreagenz, in der Regel einen Antikörper, ermöglichen.

Eine Aufgabe der vorliegenden Erfindung ist es daher, ein Reagenz zur Freisetzung von gebundenem Vitamin D zur Verfügung zu stellen, welches die bisherigen Probleme bei der Freisetzung von gebundenem Vitamin D aus Proteinkomplexen löst und für eine Verwendung in einem automatisierten Verfahren zur direkten quantitativen Bestimmung von Vitamin D geeignet ist.

Die Einschränkungen und Nachteile der Reagenzzusammensetzungen des Standes der Technik werden durch die vorliegende Erfindung zumindest teilweise behoben. Die vorliegende Erfindung stellt ein Reagenz zur Freisetzung von gebundenem Vitamin D zur Verfügung, welches eine effiziente Freisetzung von Vitamin D ermöglicht. Dieses Reagenz kann zusätzlich ein Bestimmungsreagenz von Vitamin D enthalten. Die Verwendung des Freisetzungsreagenzes bietet den Vorteil, dass das zu bestimmende Vitamin D nicht aus der Probe isoliert werden muss. Die Probe mit dem zugesetzten Freisetzungsreagenz kann ohne zusätzliche Behandlung direkt einem immunologischen Bestimmungsverfahren unterzogen werden. Dies ermöglicht eine kostengünstigere und zeitsparendere Bestimmung von Vitamin D als im Stand der Technik bekannt.

Durch die vorliegende Erfindung wird somit ein zuverlässiges und automatisierbares Verfahren zur Bestimmung der Konzentration von Vitamin D, insbesondere von 25-Hydroxyvitamin D3 oder 25-Hydroxyvitamin D2, bereitgestellt.

Ein erster Aspekt der vorliegenden Erfindung betrifft daher ein *in vitro* Verfahren zur Freisetzung von Vitamin D, umfassend die Schritte:
a) Inkontaktbringen einer Probe, die gebundenes Vitamin D, insbesondere in Form von Komplexen mit Proteinen, enthält, mit einem Freisetzungsreagenz, umfassend:
   i. mindestens eine hydrotrope Substanz, und
   ii. mindestens ein Übergangsmetallsalz,
   und
b) Freisetzen des gebundenen Vitamin D.

Wenn nicht anders angegeben, schließt der Ausdruck "*Vitamin D*" alle natürlich vorkommenden Verbindungen ein, welche das Vitamin D2 Grundgerüst oder das Vitamin D3 Grundgerüst gemäß den Strukturformeln I oder II aufweisen.

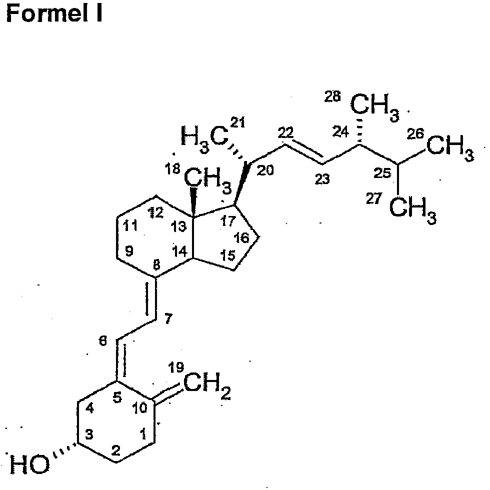

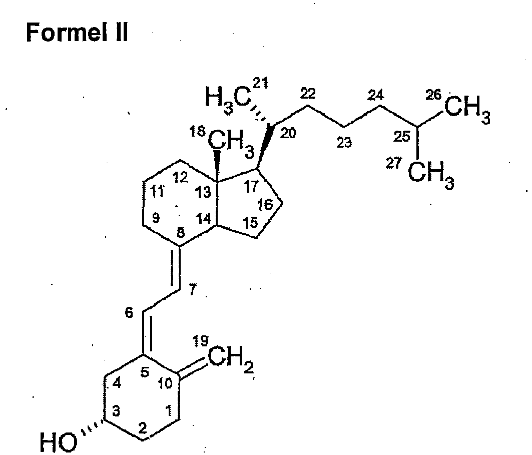

Die Nummerierung der Kohlenstoffatome in den Strukturformeln I und II ist gemäß der Steroid-Nomenklatur angegeben. 25-Hydroxyvitamin D bezeichnet Vitamin D Metaboliten, welche an Position 25 der Strukturformeln I oder II hydroxyliert sind, d.h. 25-Hydroxyvitamin D2 sowie 25-Hydroxyvitamin D3. Weitere Hydroxyvitamin D-Verbindungen sind beispielsweise 1,25-Dihydroxyvitamin D oder 24,25-Dihydroxyvitamin D.

1,25-Dihydroxyvitamin D bezieht sich auf die aktiven Formen von Vitamin D (die sogenannten D Hormone), welche an Position 1 sowie an Position 25 der Strukturformeln I und II hydroxyliert sind.

Andere gut bekannte Vitamin D-Verbindungen sind 24,25-Dihydroxyvitamin D2, 24,25-Dihydroxyvitamin D3 und C3-epi-25-Hydroxyvitamin D.

Der Ausdruck "*Freisetzung von gebundenem Vitamin D*" bedeutet die vollständige oder teilweise Abtrennung von Vitamin D aus Komplexen mit Proteinen, insbesondere DBP, an welche es gebunden ist. Vorzugsweise wird im Wesentlichen das gesamte in der Probe vorhandene Vitamin D freigesetzt. In diesem Zusammenhang bedeutet "*im Wesentlichen*", dass mindestens 90%, 95%, 98% und bevorzugt mindestens 99% des Vitamin D freigesetzt wird.

Der Ausdruck "*hydrotrope Substanz*" oder "*hydrotrope Verbindung*" bezeichnet Verbindungen, welche die Löslichkeit von unlöslichen oder schwer löslichen organischen Verbindungen in wässrigen Medien erhöhen. Dabei vermindern die hydrotropen Verbindungen die Oberflächenspannung des Wassers und erleichtern somit die Dispergierung der zu lösenden Stoffe (Hydrotropie).

Der Ausdruck "*Übergangsmetallsalz*" bezeichnet anorganische oder organische Salze von Metallen der Gruppen III-XII beziehungsweise der Gruppen Ib-VIIb und VIII des Periodensystems der Elemente.

Die zu untersuchende Probe ist vorzugsweise eine biologische Flüssigkeit, die z.B. ausgewählt ist aus Blut, Serum, Plasma oder Milch. Die Probe stammt üblicherweise von einem Lebewesen, vorzugsweise von einem Säugetier, z.B. von einem Menschen.

Die hydrotrope Substanz ist vorzugsweise eine aromatische Säure und/oder ein Ester, Amid oder Salz davon. Bevorzugt wird diese ausgewählt aus der Gruppe bestehend aus N,N-Dimethylbenzamid, Dimethylbenzoesäure, 1,2-Naphthochinonsulfonsäure, para-Toluolsulfonsäure, Sulfanilsäure, Anthrachinon-2-sulfonsäure, Anilin-2-sulfonsäure und/oder Salzen davon. Besonders bevorzugt ist die hydrotrope Substanz Toluolsulfonsäure und/oder ein Ester, Amid oder Salz davon.

Die hydrotrope Substanz weist weiterhin bevorzugt einen HLB-Wert von 12-18 auf. Der Ausdruck "*HLB-Wert*" (hydrophilic lipophilic balance) ist ein von W. C. Griffin eingeführtes Maß der Wasser- und Fettfreundlichkeit von nicht ionogenen Tensiden und Emulgatoren. Der entsprechende HLB-Wert einer Verbindung kann durch im Fachgebiet bekannte Methoden ermittelt werden.

Die Übergangsmetallsalze des erfindungsgemäßen Verfahrens umfassen bevorzugt Salze der Metalle aus den Gruppen 5, 6, 7, 8, 10 und/oder 12, z.B. Salze von Vanadium, Chrom, Mangan, Eisen, Cobalt, Nickel und/oder Zink. Besonders bevorzugt ist das Übergangsmetallsalz ein Eisen(III) Salz und am meisten bevorzugt Eisen(III)chlorid oder Eisen(III)citrat. Die Salze bestehen dabei aus Übergangsmetallkationen und anorganischen oder organischen Anionen.

Die anorganischen Anionen umfassen Halogenide, wie beispielsweise Fluorid, Chlorid, Bromid oder Iodid, Sulfid, Carbonat, Hydrogencarbonat, Sulfat, Phosphat, Nitrat und Rhodanid.

Die organischen Anionen umfassen Carbonsäureanionen, wie beispielsweise Formiat, Acetat, Palmitat, Citrat, Oxalat, Fumarat, Benzoat, Maleat oder Salicylat, und organische Sulfate, wie beispielsweise Laurylsulfat.

Das Freisetzungsreagenz kann weiterhin einen Komplexbildner, bevorzugt Citrat, EDTA und/oder ein Salz davon, am meisten bevorzugt Citrat und/oder ein Salz davon, enthalten. Der Ausdruck "*Komplexbildner*" bezeichnet Verbindungen, die zur Bildung von Komplexen befähigt sind, wie beispielsweise Chelatbildner.

Das Freisetzungsreagenz wird der Probe vorzugsweise in einer Menge zugesetzt, um eine Endkonzentration von 200 bis 1000 mM, insbesondere von 300 bis 700 mM, der hydrotropen Substanz und eine Endkonzentration von 5 bis 100 mM, insbesondere von 10 bis 40 mM, des Übergangsmetallsalzes einzustellen. Das Freisetzungsreagenz wird vorzugsweise als Stammlösung in einem wässrigen Medium vorgelegt, welches 5-30 Gewichtsprozent, bevorzugt 10-25 Gewichtsprozent, der hydrotropen Substanz und 0,1-5 Gewichtsprozent, bevorzugt 0,2-2 Gewichtsprozent, des Übergangsmetallsalzes, bezogen auf das Gesamtgewicht des Freisetzungsreagenzes, aufweist.

Ein weiterer Aspekt der vorliegenden Erfindung umfasst ein *in vitro* Verfahren zum Bestimmen von Vitamin D-Verbindungen, umfassend die Schritte:
a) Inkontaktbringen einer Probe, die gebundenes Vitamin D, insbesondere in Form von Komplexen mit Proteinen, enthält, mit einem Freisetzungsreagenz, umfassend:
   i. mindestens eine hydrotrope Substanz, und
   ii. mindestens ein Übergangsmetallsalz,
b) Freisetzen des gebundenen Vitamin D, und
c) Bestimmen des freigesetzten Vitamin D.

Die Bestimmung des freigesetzten Vitamin D gemäß Schritt c) kann zeitlich nach den Schritten a) und b) oder gemeinsam mit den Schritten a) und b) durchgeführt werden. Vorzugsweise wird Schritt c) ohne Abtrennung des Freisetzungsreagenzes von dem zu bestimmenden Vitamin D gleichzeitig mit den Schritten a) und b) ausgeführt, wodurch ein Ein-Schritt-Verfahren zur Bestimmung von Vitamin D in einer Probe bereitgestellt wird.

Die Bestimmung der Konzentration des Vitamin D in Schritt c) ist bevorzugt ein immunologisches Verfahren, bei dem das zu bestimmende Vitamin D an einen immunologischen Bindungspartner bindet, um einen Immunkomplex zu bilden. Der Ausdruck "*immunologischer Bindungspartner*" schließt Antikörper ein.

Beispiele für geeignete immunologische Verfahren schließen heterogene Assays, homogene Assays, Sandwich-Assays, Kompetitionsassays, Enzym-Immun-Assays, Radioimmunassays, Fluoreszenzpolarisationsimmunassays, Mikropartikelenzymimmunassays und chemolumineszente magnetische Immunassays ein. Viele der oben beschriebenen Testformate können entweder in einem Ein-Schritt-Verfahren oder in einem Zwei-Schritt-Verfahren durchgeführt werden. Bevorzugt werden Testformate verwendet, die in einem Ein-Schritt-Verfahren durchgeführt werden können.

Der Ausdruck "*heterogener Assay*" bezieht sich auf Verfahren, in welchen die Trennung des Immunkomplexes von anderen Bestandteilen, beispielsweise durch Waschen, erforderlich ist.

Der Ausdruck "*homogener Assay*" bezieht sich auf Verfahren, in welchen der Immunkomplex nicht von den anderen Bestandteilen abgetrennt werden muss.

Der Ausdruck "*Kompetitionsassay*" bezieht sich auf Verfahren, in welchen das in der Probe vorliegende Vitamin D mit einem detektierbaren (z.B. markierten) Tracer um einen immunologischen Bindungspartner konkurriert.

Der Ausdruck "*Sandwich-Assay*" bezieht sich auf Verfahren, in welchen Vitamin D zwischen zwei immunologischen Bindungspartnern gebunden wird, von denen mindestens einer detektierbar ist (z.B. durch Markierung).

Der Ausdruck "E*nzym-Immun-Assay*" bezieht sich auf Verfahren, welche ein Enzym als Markierung nutzen. Beispiele für solche Markierungen sind alkalische Phosphatase, Meerrettichperoxidase und β-Galactosidase. ELISA (enzyme-linked immunosorbant assay) ist beispielsweise ein bevorzugter heterogener Sandwich-Enzym-Immun-Assay.

Der Ausdruck "*Radioimmunassay*" bezieht sich auf Verfahren, welche ein radioaktives Isotop als Markierung nutzen. Ein Beispiel für eine solche Markierung ist Iod 125.

Die geeigneten Verfahren umfassen einen Detektionsschritt. Dieser kann eine Markierungsdetektierung umfassen. Alle im Fachgebiet für solche Zwecke bekannten Markierungen können dabei verwendet werden. Beispiele für geeignete Markierungen schließen Enzymmarkierungen (wie oben beschrieben), Radioisotop-Markierungen (wie oben beschrieben), Fluoreszenzmarkierungen, wie zum Beispiel Fluorescein oder Rhodamin, chemolumineszierende Markierungen, wie zum Beispiel Luminol oder Acridiniumester, und Polyhistidin-Markierungen ein.

Besonders bevorzugt wird ein kompetitives immunologisches Verfahren für die Bestimmung der Konzentration von Vitamin D in Schritt c) verwendet. Geeignete kompetitive Testformate sind dem Fachmann wohl bekannt. In einem typischen kompetitiven Bindungsassay wird ein Rezeptor oder Antikörper mit einem Tracer, d.h. einer markierten Form von Vitamin D, mit der zu untersuchenden Probe in Kontakt gebracht. Die Menge an Tracer, welcher an den Rezeptor oder Antikörper gebunden vorgefunden wird, ist dann indikativ für den Anteil an unmarkiertem Vitamin D in der Probe. Alternativ kann ein kompetitiver Bindungsassay das Inkontaktbringen eines an einen Tracer gebundenen Rezeptors oder Antikörpers mit der zu untersuchenden Probe und das Messen der Menge an verdrängtem Tracer, welche indikativ für die Menge an Vitamin D in der Probe ist, umfassen. Am meisten bevorzugt wird ein mit Biotin markierter Tracer und eine Detektion durch Peroxidase-markiertes Streptavidin verwendet.

Vorzugsweise werden zur Bestimmung des Vitamin D keine Probenbestandteile abgetrennt. Die Verfahrensschritte a), b) und c) können in einem einzigen Reaktionsgefäß durchgeführt werden, ohne dass eine Extraktion oder Aufreinigung zwischen den einzelnen Verfahrensschritten erfolgt.

Das durch das erfindungsgemäße Verfahren freigesetzte und zu bestimmende Vitamin D umfasst alle bekannten Vitamin D-Verbindungen, z.B. 25-Hydroxyvitamin D3, 25-Hydroxyvitamin D2, 1,25-Dihydroxyvitamin D3, 1,25-Dihydroxyvitamin D2, Vitamin D3, Vitamin D2 und Mischungen davon. Besonders bevorzugt sind die Vitamin D-Verbindungen 25-Hydroxyvitamin D3 und/oder 25-Hydroxyvitamin D2.

Ein weiterer Aspekt der vorliegenden Erfindung betrifft ein Reagenz zum Freisetzen von Vitamin D, umfassend:
a) mindestens eine hydrotrope Substanz, und
b) mindestens ein Übergangsmetallsalz, wobei das Reagenz 5-30 Gew.-% bevorzugt 10-25 Gew.-% der mindestens einen hydrotropen Substanz und 0,1-5 Gew.-%, bevorzugt 0,2-2 Gew.-% des mindestens einen Übergangsmetallsalzes bezogen auf das Gesamtgewicht des Reagenzes in einer wässrigen Flüssigkeit aufweist.

Vorzugsweise enthält das Reagenz weiterhin einen Komplexbildner. Als Komplexbildner wird bevorzugt Citrat, EDTA und/oder ein Salz davon verwendet, bevorzugter Citrat und/öder ein Salz davon.

In einer weiteren Ausführungsform umfasst das Reagenz weiterhin mindestens ein Reagenz zur Bestimmung von Vitamin D.

Ein weiterer Aspekt der vorliegenden Erfindung ist die Verwendung des oben beschriebenen Reagenzes zur Freisetzung von gebundenem Vitamin D und gegebenenfalls zur Bestimmung des freigesetzten Vitamin D.

Im Weiteren soll die vorliegende Erfindung detaillierter durch die folgenden Zeichnungen und Beispiele beschrieben werden.

### Figurerilegende:

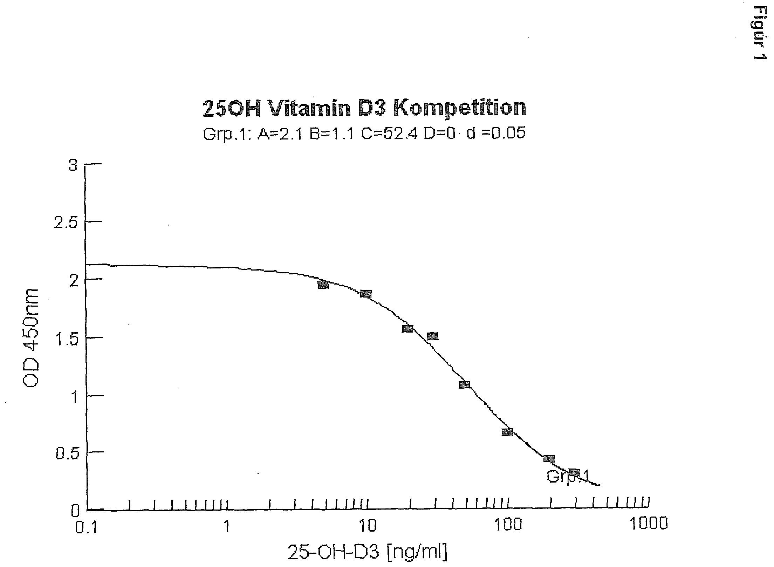

**Abb. 1****:** Charakterisierung der Freisetzung definierter 25OHD3 Konzentrationen aus der Bindung mit Serum-DBP und Kompetition mit Biotin-25OHD3 Tracerreagenz. Zur ELISA Detektion der spezifischen 25OHD Kompetition wurden Verdünnungsserien von 25OHD3 in Vitamin D-freier Serummatrix (SerCon, Seracare Inc.) in den Konzentrationen 0, 5, 10, 20, 20, 50, 100, 200, 300 ng/ml in Gegenwart von 7,5 ng/ml Biotin-25OHD Tracerreagenz mit 80 µl Vitamin D Freisetzungsreagenz versetzt und auf Mikrotiterplattenkavitäten, die mit einem Anti 25OHD Antikörper beschichtet waren, aufgetragen. Die Bindung des Tracers in Abhängigkeit der kompetierenden 25OHD Konzentration wurde mit Peroxidase-markiertem Streptavidin und einer Tetramethylbenzidin (TMB)-Farbreaktion durch Messung der OD 450 nm bestimmt.
**Abb**. **2:** Die vergleichende Analyse von Seren mit definierten 250HD3 und 25OHD2 Konzentrationen (6PLUS1 Multilevel Serum Calibrator Set 25-OH-Vitamin D3/D2, Chromsystems GmbH) und deren Verdünnungen in Vitamin D-freier Serummatrix (SerCon, Seracare Inc.) zeigte eine hohe Übereinstimmung zwischen den HPLC und LC-MS/MS definierten 25-OHD3/D2 Konzentrationen und dem direkten 25-OH Vitamin D3/D2 Bestimmungsverfahren im automatischen Aleria™ System (ORG270, ORGENTEC).

### Beispiel 1: Herstellung eines Vitamin D Freisetzungsreagenzes

Natriumtoluolsulfonat wurde in Wasser gelöst und mit Stammlösungen von 1 M Natriumcitrat und 1 M Eisen(III)chlorid auf eine Endkonzentration von 1 M Natriumtoluolsulfonat, 100 mM Natriumcitrat und 50 mM Eisen(III)chlorid eingestellt.

### Beispiel 2: Bindung von einem 25-OHD Antikörper an eine feste Phase

Ein Antikörper gegen 25-OH-Vitamin-D3 bzw. 25-OH-Vitamin-D2 wurde in Tris-Salzpuffer pH 8,0 auf eine Konzentration von 1 µg/ml verdünnt. Die Kavitäten einer Mikrotiterplatte (Maxisorb, Nunc) wurden mit 100 µl der Antikörperverdünnung beschichtet, bei 37°C getrocknet und bis zur Testdurchführung bei 4°C gelagert.

### Beispiel 3: Kompetitive Bindungsanalyse

Vitamin D-freie Serummatrix (Seracon, Seracäre Inc.) wurde mit definierten Konzentrationen 25-OHD3 versetzt. Jeweils 80 µl der Konzentrationsserie wurden in eine mit 25-OHD-Biotintracer vorbelegte Mikrotiterplattenkavität gefüllt und zur Resolubilisation des Tracerreagenz 5 min bei RT (20-27°C) inkubiert. Anschließend wurden 80 µl des Vitamin D Freisetzungsreagenzes aus Beispiel 1 der Probe zugefügt und gemischt. 100 µl der mit Vitamin D Freisetzungsreagenz verdünnten Proben wurden in 25OHD-Antikörper beschichtete Kavitäten transferiert und 30 min bei Raumtemperatur inkubiert. Anschließend wurde der Probenansatz entfernt und die Kavitäten wurden 3 X mit je 200 µl Waschpuffer gespült. Die Detektion des antikörpergebundenen Biotin-25OHD Tracers erfolgte mit Peroxidase-markiertem Streptavidin und einer Tetramethylbenzidin (TMB)-Farbreaktion. Nach Abstoppen der Substratreaktion durch Zusatz von 100 µl 50 mM Phosphorsäure erfolgte die Messung der optischen Dichte bei 450 nm. Hierbei wurde, wie in **Figur 1** dargestellt, bereits bei einer 25OHD3 Konzentration von 5 ng/ml eine spezifische Kompetition des Biotin-25OHD Tracers nachgewiesen.

### Beispiel 4: Bestimmung von 25-Hydroxyvitamin-D in Serum oder Plasma in dem automatischen Alegria™ 25-OH Vitamin D3/D2 Testsystem

Zur Untersuchung der Korrelation zwischen HPLC + LC-MS/MS basierten 25OHD3/D2 Bestimmungsverfahren und dem automatischen Alegria™ 25-OH Vitamin D3/D2 Testsystem wurden Serum- oder Plasmaproben, deren 25OHD3/D2 Konzentration über HPLC + LC-MS/MS basierte Verfahren charakterisiert wurde (6PLUS1 Multilevel Serum Calibrator Set 25-OH-Vitamin D3/D2, Chromsystems GmbH), sowie Verdünnungsstufen hieraus bis zur Bestimmung aliquotiert bei -20°C gelagert.

Zur Bestimmung im automatischen Alegria™ 25-OH Vitamin D3/D2 Testsystem wurden je 80 µl der Proben in Kavität A des Alegria™-Teststreifens vorgelegt. Die 8 Kavitäten eines Teststreifens enthielten hierbei das Vitamin D Freisetzungsreagenz, Streptavidin-HRP Konjugat, TMB-Substrat, eine 250HD Kalibratorlösung und 25-OHD Antikörper beschichtete Kavitäten für jeweils eine Probenbestimmung. Die Testbearbeitung erfolgte automatisch im Alegria™ Automaten. Hierbei wurden innehalb eines Teststreifens jeweils die Probe und der teststreifeninterne Kalibrator mit 25OHD-Biotintracer und Vitamin D Freisetzungsreagenz gemischt, in die 25OHD Antikörper beschichteten Kavitäten transferiert und nach 30 minütiger Inkubation gewaschen. Die Detektion des antikörpergebundenen Biotin-25OHD Tracers erfolgte mit Peroxidase-markiertem Steptavidiri und einer Tetramethylbenzidin (TMB)-Farbreaktion durch Messung der optischen Dichte bei 650 nm.

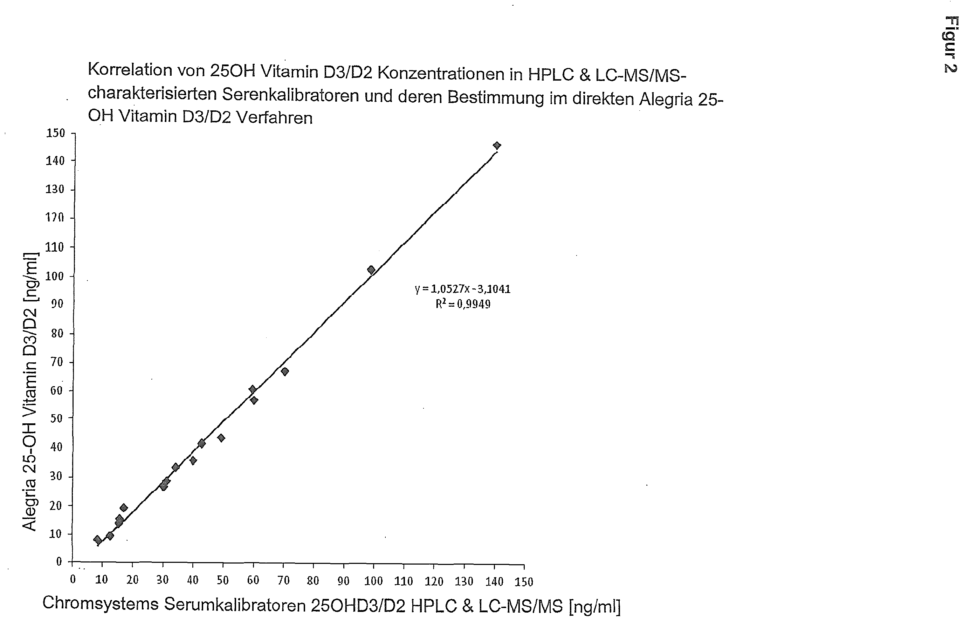

Wie in **Figur 2** dargestellt, wurde eine hohe Übereinstimmung zwischen den HPLC und LC-MS/MS definierten 25-OHD3/D2 Konzentrationen in Serenproben und dem direkten 25-OH Vitamin D3/D2 Bestimmungsverfahren im automatischen Alegria™ System (ORG270, ORGENTEC) beobachtet.

## Patentansprüche

1. In vitro Verfahren zur Freisetzung von Vitamin D, umfassend die Schritte:
a) Inkontaktbringen einer Probe, die gebundenes Vitamin D enthält, mit einem Freisetzungsreagenz, umfassend:
i. mindestens eine hydrotrope Substanz, und
ii. mindestens ein Übergangsmetallsalz,
und
b) Freisetzen des gebundenen Vitamin D.

2. Verfahren nach Anspruch 1, **dadurch gekennzeichnet, dass** die zu untersuchende Probe eine biologische Flüssigkeit ist, die bevorzugt ausgewählt wird aus Blut, Serum, Plasma oder Milch.

3. Verfahren nach Anspruch 1 oder 2, **dadurch gekennzeichnet, dass** die hydrotrope Substanz eine aromatische Säure und/oder ein Ester, Amid oder Salz davon, bevorzugt N,N-Dimethylbenzamid, Dimethylbenzoesäure, 1,2-Naphthochinonsulfonsäure, Toluolsulfonsäure, Sulfanilsäure, Anthrachinon-2-sulfonsäure, Anilin-2-sulfonsäure und/oder ein Salz davon, und besonders bevorzugt para-Toluolsulfonsäure und/oder ein Ester, Amid oder Salz davon, ist.

4. Verfahren nach einem der Ansprüche 1 bis 3, **dadurch gekennzeichnet, dass** das Übergangsmetallsalz das Salz eines Metalls aus Gruppe 5, 6, 7, 8, 10 und/oder 12, bevorzugt ein Salz von Vanadium, Chrom, Mangan, Eisen, Kobalt, Nickel und/oder Zink, besonders bevorzugt ein Eisen(III) Salz, und am meisten bevorzugt Eisen(III)chlorid oder Eisen(III)citrat, ist.

5. Verfahren nach einem der Ansprüche 1 bis 4, **dadurch gekennzeichnet, dass** das Freisetzungsreagenz weiterhin einen Komplexbildner, bevorzugt Citrat, EDTA und/oder ein Salz davon, enthält.

6. Verfahren nach einem der Ansprüche 1 bis 5, **dadurch gekennzeichnet, dass** das Freisetzungsreagenz der Probe in einer Menge zugesetzt wird, um eine Endkonzentration von 200 bis 1000 mM der hydrotropen Substanz und/oder eine Endkonzentration von 5 bis 100 mM des Übergangsmetallsalzes einzustellen.

7. In vitro Verfahren zum Bestimmen von Vitamin D, umfassend die Schritte:
a) und b) gemäß dem Verfahren nach einem der Ansprüche 1-6 zum Freisetzen von gebundenem Vitamin D, und
c) Bestimmen des freigesetzten Vitamin D.

8. Verfahren nach einem der Ansprüche 1 bis 7, **dadurch gekennzeichnet, dass** das Vitamin D ausgewählt wird aus der Gruppe umfassend 25-Hydroxyvitamin D3, 25-Hydroxyvitamin D2, 1,25-Dihydroxyvitamin D3, 1,25-Dihydroxyvitamin D2, Vitamin D3, Vitamin D2 und Mischungen davon, wobei das Vitamin D bevorzugt 25-Hydroxyvitamin D3 und/oder 25-Hydroxyvitamin D2 ist.

9. Verfahren nach einem der Ansprüche 1 bis 8, **dadurch gekennzeichnet, dass** die Bestimmung von Vitamin D ohne Abtrennung des Freisetzungsreagenzes erfolgt.

10. Verfahren nach einem der Ansprüche 1 bis 9, **dadurch gekennzeichnet, dass** die Bestimmung der Konzentration des Vitamin D in Schritt c) durch ein immunologisches Verfahren, bevorzugt durch ein kompetitives immunologisches Verfahren, erfolgt.

11. Reagenz zur Freisetzung von Vitamin D, umfassend:
a) mindestens eine hydrotrope Substanz,
b) mindestens ein Übergangsmetallsalz, und
c) gegebenenfalls mindestens einen Komplexbildner,
wobei das Reagenz 5-30 Gew.-% bevorzugt 10-25 Gew.-% der mindestens einen hydrotropen Substanz und 0,1-5 Gew.-%, bevorzugt 0,2-2 Gew.-% des mindestens einen Übergangsmetallsalzes bezogen auf das Gesamtgewicht des Reagenzes in einer wässrigen Flüssigkeit aufweist.

12. Reagenz nach Anspruch 11, **dadurch gekennzeichnet, dass** die hydrotrope Substanz eine aromatische Säure und/oder ein Ester, Amid oder Salz davon, bevorzugt N,N-Dimethylbenzamid, Dimethylbenzoesäure, 1,2-Naphthochinonsulfonsäure, para-Toluolsulfonsäure, Sulfanilsäure, Anthrachinon-2-sulfonsäure, Anilin-2-sulfonsäure und/oder Salze davon, und besonders bevorzugt Toluolsulfonsäure und/oder ein Ester, Amid oder Salz davon, ist.

13. Reagenz nach Anspruch 11, **dadurch gekennzeichnet, dass** das Übergangsmetallsalz das Salz eines Metalls aus Gruppe 5, 6, 7, 8, 10 und/oder 12, bevorzugt ein Salz von Vanadium, Chrom, Mangan, Eisen, Kobalt, Nickel und/oder Zink, besonders bevorzugt ein Eisen(III) Salz, und am meisten bevorzugt Eisen(III)chlorid oder Eisen(III)citrat, ist.

14. Reagenz zur Bestimmung von Vitamin D, umfassend ein Freisetzungsreagenz nach einem der Ansprüche 12 bis 13 und ein Bestimmungsreagenz für Vitamin D.

15. Verwendung eines Reagenzes nach einem der Ansprüche 11 bis 14 zur Freisetzung von gebundenem Vitamin D, insbesondere in einem Verfahren nach einem der Ansprüche 7 bis 10.

## Claims

1. In vitro method for releasing vitamin D, comprising the steps of:
a) bringing a sample that contains bound vitamin D into contact with a release reagent comprising:
i. at least one hydrotropic substance, and
ii. at least one transition metal salt, and
b) releasing the bound vitamin D.

2. Method according to claim 1, **characterised in that** the sample to be tested is a biological fluid which is preferably selected from blood, serum, plasma or milk.

3. Method according to either claim 1 or claim 2, **characterised in that** the hydrotropic substance is an aromatic acid and/or an ester, amide or salt thereof, preferably N,N-dimethylbenzamide, dimethylbenzoic acid, 1,2-naphthoquinonesulfonic acid, toluenesulfonic acid, sulfanilic acid, anthraquinone-2-sulfonic acid, aniline-2-sulfonic acid and/or a salt thereof, and particularly preferably para-toluenesulfonic acid and/or an ester, amide or salt thereof.

4. Method according to any one of claims 1 to 3, **characterised in that** the transition metal salt is the salt of a metal of group 5, 6, 7, 8, 10 and/or 12, preferably a salt of vanadium, chromium, manganese, iron, cobalt, nickel and/or zinc, particularly preferably an iron(III) salt, and most preferably iron(III) chloride or iron(III) citrate.

5. Method according to any one of claims 1 to 4, **characterised in that** the release reagent further contains a complexing agent, preferably citrate, EDTA and/or a salt thereof.

6. Method according to any one of claims 1 to 5, **characterised in that** the release reagent is added to the sample in an amount to give a final concentration of from 200 to 1000 mM of the hydrotropic substance and/or a final concentration of from 5 to 100 mM of the transition metal salt.

7. In vitro method for determining vitamin D, comprising the steps of:
a) and b) according to the method according to any one of claims 1 to 6 for releasing bound vitamin D, and
c) determining the released vitamin D.

8. Method according to any one of claims 1 to 7, **characterised in that** the vitamin D is selected from the group comprising 25-hydroxy vitamin D3, 25-hydroxy vitamin D2, 1,25-dihydroxy vitamin D3, 1,25-dihydroxy vitamin D2, vitamin D3, vitamin D2 and mixtures thereof, the vitamin D preferably being 25-hydroxy vitamin D3 and/or 25-hydroxy vitamin D2.

9. Method according to any one of claims 1 to 8, **characterised in that** vitamin D is determined without separating off the release reagent.

10. Method according to any one of claims 1 to 9, **characterised in that** the concentration of vitamin D is determined in step c) by an immunological method, preferably by a competitive immunological method.

11. Reagent for releasing vitamin D, comprising:
a) at least one hydrotropic substance,
b) at least one transition metal salt, and
c) optionally at least one complexing agent.
wherein the reagent has from 5 to 30 wt.%, preferably from 10 to 25 wt.%, of the at least one hydrotropic substance and from 0.1 to 5 wt.%, preferably from 0.2 to 2 wt.%, of the at least one transition metal salt, based on the total weight of the reagent, in an aqueous fluid.

12. Reagent according to claim 11, **characterised in that** the hydrotropic substance is an aromatic acid and/or an ester, amide or salt thereof, preferably N,N-dimethylbenzamide, dimethylbenzoic acid, 1,2-naphthoquinonesulfonic acid, para-toluenesulfonic acid, sulfanilic acid, anthraquinone-2-sulfonic acid, aniline-2-sulfonic acid and/or salts thereof, and particularly preferably toluenesulfonic acid and/or an ester, amide or salt thereof.

13. Reagent according to claim 11, **characterised in that** the transition metal salt is the salt of a metal of group 5, 6, 7, 8, 10 and/or 12, preferably a salt of vanadium, chromium, manganese, iron, cobalt, nickel and/or zinc, particularly preferably an iron(III) salt, and most preferably iron(III) chloride or iron(III) citrate.

14. Reagent for determining vitamin D, comprising a release reagent according to any one of claims 12 to 13 and a determination reagent for vitamin D.

15. Use of a reagent according to any one of claims 11 to 14 for releasing bound vitamin D, in particular in a method according to any one of claims 7 to 10.

## Revendications

1. Procédé in vitro pour la libération de la vitamine D, comprenant les étapes suivantes :
a) la mise en contact d'un échantillon, qui contient de la vitamine D liée, avec un réactif de libération, comprenant :
i. au moins une substance hydrotrope ; et
ii. au moins un sel de métal de transition ; et
b) la libération de la vitamine D liée.

2. Procédé selon la revendication 1, **caractérisé en ce que** l'échantillon à analyser est un liquide biologique, qui est choisi de préférence parmi le sang, le sérum, le plasma ou le lait.

3. Procédé selon la revendication 1 ou 2, **caractérisé en ce que** la substance hydrotrope est un acide aromatique et/ou un ester, un amide ou un sel de celui-ci, de préférence le N,N-diméthyl benzamide, l'acide diméthylbenzoïque, l'acide 1,2-naphtoquinone sulfonique, l'acide toluènesulfonique, l'acide sulfanilique, l'acide anthraquinone 2-sulfonique, l'acide aniline 2-sulfonique et/ou un sel de ceux-ci, et en particulier de préférence l'acide paratoluènesulfonique et/ou un ester, un amide ou un sel de celui-ci.

4. Procédé selon l'une des revendications 1 à 3, **caractérisé en ce que** le sel de métal de transition est le sel d'un métal du groupe 5, 6, 7, 8, 10 et/ou 12, de préférence un sel de vanadium, de chrome, de manganèse, de fer, de cobalt, de nickel et/ou de zinc, en particulier de préférence un sel de fer (III), et de manière préférée entre toutes un chlorure de fer (III) ou un citrate de fer (III).

5. Procédé selon l'une des revendications 1 à 4, **caractérisé en ce que** le réactif de libération contient en outre un agent de chélation, de préférence un citrate, l'EDTA et/ou un sel de celui-ci.

6. Procédé selon l'une des revendications 1 à 5, **caractérisé en ce que** le réactif de libération de l'échantillon est ajouté dans une quantité, pour ajuster la concentration finale de la substance hydrotrope à de 200 à 1000 mM et/ou la concentration finale du sel de métal de transition à de 5 à 100 mM.

7. Procédé in vitro pour la détermination de la vitamine D, comprenant les étapes suivantes :
a) et b) conformément au procédé selon l'une des revendications 1 à 6 pour la libération de la vitamine D liée ; et
c) la détermination de la vitamine D libérée.

8. Procédé selon l'une des revendications 1 à 7, **caractérisé en ce que** la vitamine D est sélectionnée dans le groupe comprenant la 25-hydroxyvitamine D3, la 25-hydroxyvitamine D2, la 1,25-dihydroxyvitamine D3, la 1,25-dihydroxyvitamine D2, la vitamine D3, la vitamine D2 et des mélanges de ces vitamines, selon lequel la vitamine D est de préférence la 25-hydroxyvitamine D3 et/ou la 25-hydroxyvitamine D2.

9. Procédé selon l'une des revendications 1 à 8, **caractérisé en ce que** la détermination de la vitamine D est réalisée sans séparation du réactif de libération.

10. Procédé selon l'une des revendications 1 à 9, **caractérisé en ce que** la détermination de la concentration de la vitamine D dans l'étape c) est réalisée par un procédé immunologique, de préférence par un procédé immunologique compétitif.

11. Réactif pour la libération de la vitamine D, comprenant :
a) au moins une substance hydrotrope ;
b) au moins un sel de métal de transition ; et
c) le cas échéant au moins un agent de chélation ;
selon lequel le réactif présente de 5 à 30 % en poids, de préférence de 10 à 25 % en poids de la au moins une substance hydrotrope et de 0,1 à 5 % en poids, de préférence une proportion comprise entre 0,2 et 2 % en poids du au moins un sel de métal de transition, par rapport au poids total du réactif dans un liquide aqueux.

12. Réactif selon la revendication 11, **caractérisé en ce que** la substance hydrotrope est un acide aromatique et/ou un ester, un amide ou un sel de celui-ci, de préférence le N,N-diméthyl benzamide, l'acide diméthylbenzoïque, l'acide 1,2-naphtoquinone sulfonique, l'acide paratoluènesulfonique, l'acide sulfanilique, l'acide anthraquinone 2-sulfonique, l'acide aniline 2-sulfonique et/ou les sels de ceux-ci, et en particulier de préférence de l'acide toluène sulfonique et/ou un ester, un amide ou un sel de celui-ci.

13. Réactif selon la revendication 11, **caractérisé en ce que** le sel de métal de transition est le sel d'un métal du groupe 5, 6, 7, 8, 10 et/ou 12, de préférence un sel de vanadium, de chrome, de manganèse, de fer, de cobalt, de nickel et/ou de zinc, en particulier de préférence un sel de fer (III), et de manière préférée entre toutes un chlorure de fer (III) ou un citrate de fer (III).

14. Réactif pour la détermination de la vitamine D, comprenant un réactif de libération selon l'une des revendications 12 à 13 et un réactif de détermination pour la vitamine D.

15. Utilisation d'un réactif selon l'une des revendications 11 à 14 pour la libération de la vitamine D liée, en particulier dans un procédé selon l'une des revendications 7 à 10.
